# EUROPEAN PATENT SPECIFICATION

(11) **EP 1 802 286 B1**
(45) Date of publication and mention of the grant of the patent: **23.07.2008**
(21) Application number: 05779051.1
(22) Date of filing: 02.08.2005
(51) Int. Cl.: A61K 9/50, A61K 9/20

(54) **SUSTAINED DRUG RELEASE COMPOSITION DEMONSTRATING AN ASCENDING ZERO ORDER RELEASE PATTERN, METHODS OF MANUFACTURING SUCH A COMPOSITION**
ZUSAMMENSETZUNG MIT VERZÖGERTER ARZNEIMITTELFREISETZUNG MIT EINEM AUFSTEIGENDEN NULLORDNUNG-FREIGABE-MUSTER; VERFAHREN ZUR HERSTELLUNG EINER SOLCHEN ZUSAMMENSETZUNG"
COMPOSITION DE LIBERATION SOUTENUE DE MEDICAMENT MANIFESTANT UN SCHEMA DE LIBERATION D'ORDRE CROISSANT NUL, PROCEDES DE FABRICATION D'UNE TELLE COMPOSITION

(30) Priority: 04.08.2004 US 599126 P
(43) Date of publication of application: 04.07.2007
(73) Proprietor: ALZA CORPORATION, Mountain View, CA 94039-7210 (US)
(72) Inventor: CASADEVALL, Gemma, E-08960 Barcelona (ES); SUBRAMANIAN, Ramkumar, Mountain View, CA 94041 (US); BARCLAY, Brian, Sunnyvale, CA 94087 (US); ALLPHIN, Clark, Mountain View, CA 94043 (US); SHIVANAND, Padmaja, Los Altos, California 94022 (US); YAM, Noymi, V., Sunnyvale, CA 94086 (US)
(74) Representative: Williams, Paul Edwin
(86) International application number: PCT/US2005/027490
(87) International publication number: WO 2006/017537

(56) References cited:
- EP-A- 0 598 309
- WO-A-96/19201
- WO-A-03/007919
- US-A- 5 407 687

## Description

### FIELD OF THE INVENTION

This invention pertains to methods and devices for maintaining a desired therapeutic drug effect over a prolonged therapy period. In particular, the invention is directed to methods and devices that provide drug release within the gastrointestinal tract at an ascending release rate over an extended time period. In this manner, drug is released at an ascending rate during a portion of the drug administration period sufficient to maintain a desired therapeutic drug effect throughout a prolonged therapy period.

### BACKGROUND

To produce its pharmacological effects, a drug must be made available in appropriate concentrations at its site of action within the body. This availability is affected by numerous factors including the quantity of the drug administered, the extent and rate of its absorption from its administration site, its distribution, binding or localization within tissues, its biotransformation and its excretion. One commonly-used indicator of drug availability is the concentration of drug that is obtained within the blood or plasma, or other appropriate body fluid or tissue, of a patient following administration of the drug. For convenience, this concentration may be referred to as "plasma drug concentration" hereinafter which is intended to be inclusive of drug concentration measured in any appropriate body fluid or tissue. Plasma drug concentration measurements provide very useful information including, for example, comparative information with regard to different drug dosage forms and/or different drug administration routes. In addition, for many drugs, various drug effects including both desired pharmacological effects, i.e., therapeutic drug effects, and undesired pharmacological effects, i.e., side effects, have been correlated with specific plasma drug concentrations or ranges of plasma drug concentrations.

For orally administered drug dosage forms, absorption occurs within the gastrointestinal ("Gl") tract and is affected by many factors including the physicochemical properties of the local microenvironment, such as surface area, blood flow and membrane characteristics (which vary significantly in the different portions of the GI tract), the physicochemical properties of the drug entity, drug concentration, the existence and activity of drug-specific transport mechanisms, etc. One important factor in the rate of absorption of drug administered as an oral dosage form is the rate at which drug is released from the dosage form. Drug release rates for oral dosage forms are typically measured as an in vitro rate of dissolution, i.e., a quantity of drug released from the dosage form per unit time.

Conventional oral dosage forms can be described as "immediate-release" because, generally, essentially the entire dose of drug is released from the dosage form within a very short period, i.e., minutes, following administration. As this bolus of released drug is absorbed, the plasma drug concentration typically rapidly rises to a maximal or peak concentration and subsequently declines as the drug is distributed, bound or localized within tissues, biotransformed and/or excreted. The time period for this decline varies for different drugs and depends on many factors but this time period will be characteristic of a particular drug. Generally, during some portion of the time period in which the plasma drug concentration rises, peaks and declines, the drug provides its therapeutic effects, i.e., the plasma drug concentration achieves or exceeds an effective concentration. Moreover, at some point during this time period, the therapeutic effects disappear, i.e., when the plasma drug concentration declines to a level that is below an effective concentration. In addition, often, during a portion of this time surrounding the time the peak concentration is attained, i.e., when the plasma drug concentration is in its highest range, undesired side effects may become apparent.

In view of the above, it will be appreciated that continued drug effectiveness occurs during the time period when the plasma drug concentration is within the effective plasma drug concentration range. Because the plasma drug concentration declines over time, however, multiple doses of the immediate-release drug dosage form must be administered at appropriate intervals to ensure that the plasma drug concentration remains in or, again, rises to, the effective concentration range. At the same time, however, there is a need to avoid or minimize plasma drug concentrations that rise to, and/or that remain for too long within, the higher ranges where side effects become apparent. Accordingly, for many drugs, multiple, separate doses of the immediate-release dosage form must be administered at appropriate intervals to maintain a satisfactory balance of desired and undesired pharmacological effects over a prolonged therapy period.

One focus of efforts to improve drug therapy has been directed to providing non-immediate-release oral drug dosage forms that affect absorption of the drug primarily by altering the release rate of the drug from the dosage form. Examples of such non-immediate-release delivery systems include delayed-release and sustained-release systems. Sustained-release dosage forms generally release drug for an extended time period compared to an immediate-release dosage form. There are many approaches to achieving sustained release of drugs from oral dosage forms known in the art. These different approaches include, for example, diffusion systems such as reservoir devices and matrix devices, dissolution systems such as encapsulated dissolution systems (including, for example, "tiny time pills") and matrix dissolution systems, combination diffusion/dissolution systems, osmotic systems and ion-exchange resin systems as described in Remington's Pharmaceutical Sciences, 1990 ed., pp. 1682-1685. All references cited to herein are hereby incorporated by reference in their entirety as if reproduced herein.

It is believed to be particularly desirable to provide sustained-release oral dosage forms that provide drug release at a substantially constant release rate over an extended time period. In this manner, for many drugs, the plasma drug concentration initially ascends for a short period of time as drug release begins and then remains substantially constant over an extended time period as drug release continues at a constant rate. For many drugs, this substantially constant plasma drug concentration correlates with substantially constant drug effectiveness over a prolonged therapy period. In addition, because an initial relatively high peak plasma drug concentration is avoided, side effects may be less of a problem. Accordingly, advantages of constant-release dosage forms include decreasing the number of doses of a drug that need to be administered over time and providing a better balance of desired and undesired pharmacological effects of the drug.

Although constant-release dosage forms have proven effective for many different drug therapies, there are clinical situations where these have not been entirely satisfactory. It has been observed that for some patients being treated with constant-release dosage forms for some conditions or diseases, the therapeutic effectiveness of the drug decreases at time periods before the end of the desired therapy period despite the maintenance of substantially constant drug release that would be expected to provide continued effectiveness. Accordingly, there remains a need to provide alternative means of controlled delivery in a variety of patterns.

One such circumstance wherein sustained-release dosage forms that release drug at a substantially constant rate over an extended time period are not satisfactory has been administration of drug at a release rate that is ascending, rather than substantially constant. An example of a clinical situation where drug therapy with sustained-release oral drug dosage forms that provide a substantially constant rate of drug release for an extended period has not been entirely satisfactory is with the use of central nervous system (CNS) stimulant drugs to treat various conditions and disorders including Attention Deficit Disorder (ADD) and Attention Deficit Hyperactivity Disorder (ADHD).

Published United States Patent Application No. 20010012847 to Lam et al., discloses dosage forms possessing ascending release rate profiles useful in the treatment of such conditions and disorders.
US5407687, EP0598309, WO03/007919, WO96/19201 disclose bilayer or multiple layer pharmaceutical forms for oral administration. The layers generally comprise an active ingredient and a cellulose derivative. EP0598309 is based on a) at least one swelling layer containing one or more active principles in a matrix of swellable, hydrophilic polymers (methylcellulose, carboxy methylcellulose, hydroxypropylcellulose, hydroxypropylmethylcellulose, polyvinylpyrrolidone, polymethacrylates), b) at least one erodible and/or soluble layer comprising excipients and/or water soluble polymers, one or more active principles. WO96/19201 reveals to be specific, since the controlled-release form consists of at least one layer which comprises a beneficial agent and a polymer which forms microscopic gel beads upon hydration and further one layer which comprises a polymer forming microscopic gel beads upon hydration besides an impermeable, insoluble coating.

Additional dosage forms that provide ascending rates of release would be a useful addition to the art. Accordingly, a need arises for sustained-release matrix oral dosage forms adapted to provide such a release rate over a suitable extended time period, together with methods of making and using such dosage forms.

### SUMMARY OF THE INVENTION

In an aspect, the invention relates to a dosage form for sustained release of a drug comprising: a delay layer comprising (i) a polymeric matrix, and (ii)microencapsulated drug, wherein the delay layer is substantially free of non-microencapsulated drug; and a second layer comprising (iii) a polymeric matrix, and (iv) non-microencapsulated drug matrix; wherein the second layer is located adjacent to the delay layer.

In another aspect, the invention relates to a method of making a dosage form for sustained release of a drug comprising: providing a delay layer comprising (v)a polymeric matrix, and (vi) microencapsulated drug, wherein the delay layer is substantially free of non-microencapsulated drug; and providing a second layer comprising (vii)a polymeric matrix, and (viii) non-microencapsulated drug matrix; and locating the second layer adjacent to the delay layer.

### BRIEF DESCRIPTION OF THE DRAWINGS

Figure 1 shows a dosage form according to the invention.

Figure 2 shows a dosage form according to the invention.

Figure 3 shows a dosage form according to the invention.

Figure 4 shows a dosage form according to the invention.

Figure 5 shows a dosage form according to the invention.

Figure 6 shows a dosage form according to the invention.

Figure 7 shows release rate data.

Figure 8 shows release rate data.

Figure 9 shows a dosage form according to the invention.

Figure 10 shows release rate data.

Figure 11 shows release rate data.

Figure 12 shows the layout of a dosage form

Figure 13 shows release rate data.

### DETAILED DESCRIPTION

The inventors have unexpectedly discovered that the problems in the art as described above can be overcome by using a combination of a polymer matrix, non-microencapsulated drug and microencapsulated drug. In particular, the problems in the art may be overcome by providing a dosage form for sustained release of a drug comprising a delay layer comprising polymeric matrix, and microencapsulated drug, wherein the delay layer is substantially free of non-microencapsulated drug; and a second layer comprising a polymeric matrix, and non-microencapsulated drug matrix; wherein the second layer is located adjacent to the delay layer. In an embodiment, the invention relates to such dosage forms, wherein the ratio of the weight of microencapsulated drug present in the delay layer to the weight of non-microencapsulated drug present in the second layer ranges from 0.01:1 to 10:1

While not wishing to be limited by a particular mechanism, it appears that drug release from a dosage form according to the invention may work by a combination of diffusion and erosion. In operation, the polymeric matrix hydrates and swells due to media penetration. This creates different regions inside the polymeric matrix (dry core, gel layer and undissolved gel layer) that generates three moving fronts: a swelling front, an erosion front and a diffusion front. Interactions between water, polymer, excipient and drug are the main factors determining drug release. Addition of microencapsulated drug provides a reservoir of drug that is released more slowly than the non-microencapsulated drug because of the hydration delay introduced by the microencapsulating material. Any drug release from the dosage form would then depend on polymer relaxation, swelling and dissolution as well as drug transport across the polymeric matrix gel layer, except for the very few microencapsulated and non-microencapsulated drug particles that happen to be at the very surface of the dosage form. In an embodiment, the polymeric matrix material and microencapsulation material are selected such that the ratio of the rate of release of microencapsulated drug from the polymeric matrix is from 0.1 to 3, preferably 0.5 to 1.0, times slower than the release of non-microencapsulated drug from the polymeric matrix.

In another embodiment, additional layers, such as sub-layers, may be included in the inventive dosage forms in order to adjust the overall release rate of drug from the dosage form. For example, addition of a layer that is substantially free from, or that contains a specified amount of drug - either microencapsulated or non-microencapsulated - can delay or reduce the rate of release of the drug from the dosage form at various time during operation of the dosage form. Other variables that can affect drug release (assuming that the polymeric matrix and the microencapsulation material remain the same) include drug to polymer concentration ratio (more polymer and less drug decreases release rate), and dosage form geometry (more surface area available for release increases the release rate). With decreasing polymer molecular weight the degree of entanglement of the macromolecules decreases. Thus, the mobility of the polymer chains on water imbibition increases. This leads to increased water and drug diffusion coefficients and increased drug release rates.

Before the present invention is described in detail, it is to be understood that unless otherwise indicated this invention is not limited to specific pharmaceutical agents, excipients, polymers, salts, or the like, as such may vary. It is also to be understood that the terminology used herein is for the purpose of describing particular embodiments only and is not intended to limit the scope of the present invention.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Although the present invention is illustrated herein by exemplary dosage forms containing specific exemplary drugs, methods of making such dosage forms and methods of using topiramate, paliperidone and risperidone-containing dosage forms to provide a desired therapeutic outcome, the invention is not limited by the exemplary embodiments. The invention broadly embraces oral sustained-release dosage forms that provide an ascending drug release rate over an extended time period, methods of making such dosage forms and methods of using such dosage forms to maintain therapeutic effectiveness for a desired prolonged therapy period with respect to any appropriate drugs and drug therapies as would be apparent to a person of skill in the art in view of the disclosure herein.

The term "ascending rate of release" means a rate of release wherein the amount of drug released as a function of time increases over a period of time, preferably continuously and gradually. Preferably, the rate of drug released as a function of time increases in a steady (rather than stepwise) manner. More preferably, an ascending rate of release may be characterized as follows. The rate of release as a function of time for a dosage form is measured and plotted as % drug release versus time or as milligrams of drug released / hour versus time. An ascending rate of release is characterized by an average rate (expressed in mg of drug per hour) wherein the rate within a given two hour span is higher as compared with the previous two hour time span, over the period of time of about 2 hours to about 12 hours, preferably, about 2 hours to about 18 hours, more preferably about 4 hours to about 12 hours, more preferably still, about 4 hours to about 18 hours. Preferably, the increase in average rate is gradual such that less than about 30% of the dose is delivered during any 2 hour interval, more preferably, less than about 25% of the dose is delivered during any 2 hour interval. Preferably, the ascending release rate is maintained until at least about 50%, more preferably until at least about 75% of the drug in the dosage form has been released. In an embodiment, the ratio of the weight of non-microencapsulated drug present in the second layer to the weight of microencapsulated drug present in the delay layer is selected to achieve release of between 10% and 40% of total drug weight by about T25, release of between 40% and 65% of total drug weight by about T50, release of between 65% and 85% of total drug weight by about T75, and release of between 85% and 90% of total drug weight by about T90.

It will be appreciated that the periodic release rate measured, e.g., the periodic release rate at t=1 hour (unless equal to 0), will always be greater than the release rate during the preceding period, e.g., the hour before the dosage form was administered, and, thus, the first periodic release rate always constitutes an occurrence of an ascending release rate. The ascending release rates described in examples herein refer to both: release rate from a dosage form adapted to provide sustained release of drug and also embodiments comprising an initial immediate-release component dose of a drug additionally to the sustained release component. Thus, for this second embodiment, after an initial drug peak, the following release rate measurements would also be greater than the release rate during the preceding period.. This invention also applies to dosage form embodiments additionally comprising an immediate-release dose of a drug applied as a coating onto the underlying dosage form, the drug release measured at t=1 hour will generally reflect both the drug released from the immediate-release drug coating and any drug released from the underlying dosage form, however, the quantity of drug released from the drug overcoat is disregarded in determining whether the drug release rate at t=2 hours is greater than the drug release at t=1 hour.

As used herein with reference to the time period during which an ascending release rate is provided, "an extended time period" refers to a time period beginning at t=0 hours and continuing through at least the mid-point, and preferably beyond the mid-point, of the relevant T.sub.90 of the dosage form. Because the dosage forms of the present invention are intended to provide sustained release of drug, a suitable T.sub.90 for purposes of this invention is at least about 6 hours and, consequently, the "extended time period" during which an ascending release rate is provided is at least 3 hours.

The term "ascending drug plasma concentration" means a drug plasma concentration profile over about the first 24 hours following initial dosing, wherein the profile shows an increase to a maximum concentration, wherein said maximum occurs more than about 6 hours following the initial dose, preferably, more than about 8 hours following initial dose, more preferably, more than about 12 hours after dose.

The term "delay layer" means a layer that functions, at least in part, to retard release of the drug from the dosage form.

The terms "deliver" and "delivery" refer to separation of the pharmaceutical agent from the dosage form, wherein the pharmaceutical agent is able to dissolve in the fluid of the environment of use.

The term "dosage form" means a pharmaceutical composition or device comprising a drug, the composition or device optionally containing pharmacologically inactive ingredients, i.e., pharmaceutically acceptable excipients such as polymers, suspending agents, surfactants, disintegrants, dissolution modulating components, binders, diluents, lubricants, stabilizers, antioxidants, osmotic agents, colorants, plasticizers, coatings and the like, that are used to manufacture and deliver drugs.

The term "drug" means a pharmaceutically active agent or a pharmaceutically acceptable salt thereof. Drugs useful in the practice of this invention include, but are not limited to the following: prochlorperzine edisylate, ferrous sulfate, aminocaproic acid, mecamylamine hydrochloride, procainamide hydrochloride, amphetamine sulfate, methamphetamine hydrochloride, benzamphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, digoxin, isoflurophate, acetazolamide, methazolamide, bendroflumethiazide, chloropromaide, tolazamide, chlormadinone acetate, phenaglycodol, allopurinol, aluminum aspirin, methotrexate, acetyl sulfisoxazole, erythromycin, topiramate, paliperidone, risperidone, oxybutynin, methyl phenidate, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17-.varies.hydroxyprogesterone acetate, 19-norprogesterone, norgestrel, norethindrone, norethisterone, norethiederone, progesterone, norgesterone, norethynodrel, aspirin, acetaminophen, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, theophylline, calcium gluconate, ketoprofen, ibuprofen, cephalexin, erythromycin, haloperidol, zomepirac, ferrous lactate, vincamine, diazepam, phenoxybenzamine, diltiazem, milrinone, capropril, mando, quanbenz, hydrochlorothiazide, ranitidine, flurbiprofen, fenufen, fluprofen, tolmetin, alclofenac, mefenamic, flufenamic, difuinal, nimodipine, nitrendipine, nisoldipine, nicardipine, felodipine, lidoflazine, tiapamil, gallopamil, amlodipine, mioflazine, lisinolpril, enalapril, enalaprilat, captopril, ramipril, famotidine, nizatidine, sucralfate, etintidine, tetratolol, minoxidil, chlordiazepoxide, diazepam, amitriptyline, imipramine, and terazosine HCl di-hydrate. Further examples are proteins and peptides which include, but are not limited to, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatotropin, oxytocin, vasopressin, GRF, prolactin, somatostatin, lypressin, pancreozymin, luteinizing hormone, LHRH, LHRH agonists and antagonists, leuprolide, interferons, interleukins, growth hormones such as human growth hormone, bovine growth hormone and porcine growth hormone, fertility inhibitors such as the prostaglandins, fertility promoters, growth factors, coagulation factors, human pancreas hormone releasing factor, analogs and derivatives of these compounds, and pharmaceutically acceptable salts of these compounds or their analogs or derivatives, and various combinations of these compounds, and various combinations of these compounds with various pharmaceutically acceptable salts of the above compounds.

The term "immediate-release dosage form" means a dosage form that releases drug substantially completely within a short time period following administration, i.e., generally within a few minutes to about 1 hour.

The term "matrix" means an element of the inventive dosage form that provides support or structure, especially in the sense of surrounding and/or shaping.

The term "patient" as used herein, refers to an animal, typically a mammal, and more typically, a human, in need of treatment for a disease or disorder.

As used herein, unless otherwise noted, the term "pharmaceutically acceptable salt", shall mean any salt whose anion or cation does not contribute significantly to the toxicity or pharmacological activity of the salt, and, as such, they are the pharmacological equivalents of the acids or bases of the compound. Suitable pharmaceutically acceptable salts include acid addition salts which may, for example, be formed by reacting the drug compound with a suitable pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid; and base addition salts, including alkali metal salts, e.g., sodium or potassium salts; alkaline earth metal salts, e.g., calcium or magnesium salts; and salts formed with suitable organic ligands, e.g., quaternary ammonium salts, which may be similarly prepared by reacting the drug compound with a suitable pharmaceutically acceptable base.

Thus, representative pharmaceutically acceptable salts include, but are not limited to, the following: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide, valerate, and combinations thereof.

Representative acids and bases which may be used in the preparation of pharmaceutically acceptable salts include the following: acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, a-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide, and combinations thereof.

The term "polymer" comprises natural and synthetic polymers that, upon hydration, develop a viscous and gelatinous surface barrier or gel layer, which controls the drug release from and the liquid penetration into the centre of the dosage form. In certain embodiments, the subsequent physicochemical characteristics of this gel layer control water uptake and the drug release mechanism from the dosage form. Although a rapid burst release of soluble drugs from the external layer may occur, drug release is controlled over time either by diffusion of the drugs through the gel layer or by gradual erosion of the gel, exposing fresh surfaces containing drug to the dissolution media. Diffusion is the dominant mechanism controlling the release of water-soluble drugs, and erosion of the matrix is the dominant mechanism controlling the release of water insoluble drugs. However, generally, release of drugs will occur by a mixture of these two mechanisms.

Suitable examples of polymers include, but are not limited to: hydrophilic cellulose derivatives such as methyl cellulose, hydroxypropyl methylcellulose, hydroxyethyl cellulose, hydroxypropyl cellulose, hydroxyethyl methylcellulose, carboxy methylcellulose and sodium carboxy methylcellulose; polyvinylpyrrolidone, polyethylene oxide; and polysaccharides. A preferred polymer is hydroxypropyl methylcellulose (HPMC) and derivatives thereof.

HPMC derivatives are available in a low, normal or high viscosity grades. The viscosity of the polymer controls a release rate of the drug from the formulation. Specific HPMCs which are most suitable are Methocel K100M, K15M, F4M, E4M, K4M, K100LV, K3, E15LV, E15LN, E15CLV, E50, E5 and E3 (available from Dow Chemical, Midland MI). Most preferred is Methocel K100M.

The term "polymer and non-microencapsulated drug matrix" means a matrix that comprises polymer and non-microencapsulated drug.

The term "polymeric matrix" means a matrix that comprises polymer(s).

The term "microencapsulated drug" means drug particles that are encapsulated by microencapsulation materials. Such materials include, but are not limited to, proteins, polysaccharides, starches, waxes, fats, natural and synthetic polymers, and resins and/or combinations thereof. In a preferred embodiment, a microencapsulation material comprises synthetic polymers, more preferably ethyl acrylate-methyl methacrylate copolymer. Suitable methods for producing microencapsulated drug include, but are not limited to, fluidized bed coating, spray drying, spray chilling, spinning disk coating, stationary nozzle coextrusion, pelletization, centrifugal head coextrusion, submerged nozzle coextrusion, pan coating, solvent evaporation, coacervation (simple and complex) and phase separation, interfacial polymerization, in-situ polymerization, liposome technology and nanoencapsulation. A preferred embodiment is fluidized bed coating. Additional techniques and materials useful in the practice of this invention include, but are not limited to PJ Watts et al., Microencapsulation using emulsification/solvent evaporation: an overview of techniques and applications, Crit Rev Ther Drug Carrier Syst. 1990;7(3):235-59; United States Patent 5,362,424 to Lee, et al. granted November 8, 1994 and entitled Microencapsulation for controlled oral drug delivery system; United States Patent 5,407,609 to Tice, et al. granted April 18, 1995 and entitled Microencapsulation process and products therefrom; United States Patent 4,451,452 to Deibig, et al. granted May 29, 1984 and entitled Pharmaceutical compositions containing biodegradable polymers. Typical microencapsulated drug particles size ranges are listed below; one of skill can adjust the encapsulation method to achieve the desired size:

| PHYSICAL METHODS | |
|---|---|
| Encapsulation Method | Size Range |
| Stationary coextrusion | 1,000-6,000 |
| Centrifugal coextrusion | 125-3,000 |
| Submerged nozzle coextrusion | 700-8,000 |
| Vibrating nozzle | >150 |
| Rotating disk | 5-1,000 |
| Pan coating | >500 |
| Fluid bed | 50-10,000 |
| Spray drying | 20-150 |

| CHEMICAL METHODS | |
|---|---|
| Encapsulation Method | Size Range |
| Simple/complex coacervation | 1-500 |
| Phase separation | 1-500 |
| Interfacial polymerization | 1-500 |
| Solvent evaporation | 1-500 |
| *In situ* polymerization | 1-500 |
| Liposome | 0.1-1 |
| Sol-gel methods | 0.1-1 |
| Nanoencapsulation | <1 |

The term "non-microencapsulated drug" means a drug that is substantially not a microencapsulated drug. Non-microencapsulated drug may be located in one or in more than one layer of the inventive dosage forms. In embodiments wherein a non-microencapsulated drug is present in more than one layer, the non-microencapsulated drug may be present in the same or different concentrations in each layer. One or more than one (i.e. different, or mixtures) non-microencapsulated drugs may be present within the inventive dosage forms.

The terms "rate of release" or "release rate" of a drug means the quantity of drug released from a dosage form per unit time, e.g., milligrams of drug released per hour (mg/hr). Drug release rates for drug dosage forms are typically measured as an in vitro rate of dissolution, i.e., a quantity of drug released from the dosage form per unit time measured under appropriate conditions and in a suitable fluid. For example, dissolution tests can be performed on dosage forms placed in metal coil sample holders attached to a USP Type VII bath indexer and immersed in about 50 ml of acidified water (pH = 3) equilibrated in a constant temperature water bath at 37° C. Aliquots of the release rate solutions are tested to determine the amount of drug released from the dosage form, for example, the drug can be assayed or injected into a chromatographic system to quantify the amounts of drug released during the testing intervals.

As used herein, unless otherwise specified, a drug release rate obtained at a specified time "following administration" refers to the in vitro drug release rate obtained at the specified time following implementation of an appropriate dissolution test. The time at which a specified percentage of the drug within a dosage form has been released may be referenced as the "Tx" value, where "x" is the percent of drug that has been released. For example, a commonly used reference measurement for evaluating drug release from dosage forms is the time at which 90% of drug within the dosage form has been released. This measurement is referred to as the "T90" for the dosage form. For clarity and convenience herein, the convention is utilized of designating the time of drug administration as zero hours (t = 0 hours) and times following administration in appropriate time units, e.g., t = 30 minutes or t = 2 hours, etc.

The term "substantially free" means that the dosage form, layer, or element in question is substantially free of the material in question. In a preferred embodiment, the dosage form, layer, or element in question comprises less than about 5 wt%, more preferably less than about 2.5 wt%, and more preferably still less than about 1 wt% of the material in question, based on the total weight of the dosage form, layer, or element in question.

The term "sustained release" means the drug is released from the sustained release dosage form at such a rate that blood (e. g., plasma) concentrations (levels) are maintained within the therapeutic range but below toxic levels, over a period of time of about 12 hours or longer.

By "sustained release dosage form" is meant a dosage form that releases drug for many hours. The dosage forms in accord with the present invention exhibit T90 values of at least four hours or more and preferably up to about 24 hours or more, for once per daily (qd) dosing. The dosage forms continuously release drug for sustained periods of at least about 6 hours, preferably about 8 hours or more and, in particular embodiments, about 12 hours or more.

The term "zero order plasma profile" or "flat plasma profile" means a substantially flat or unchanging amount of a particular drug in the plasma of a patient over a particular time interval. Generally, a zero order plasma profile will vary by no more than about 10% from one time interval to the subsequent time interval.

The term "zero order release rate" means a substantially constant release rate, such that the drug dissolves in the fluid at the environment of use at a substantially constant rate. More particularly, the rate of release of drug as a function of time shall vary by less than 30%, preferably, less than 20%, more preferably, less than 10%, most preferably, less than 5%, wherein the measurement is taken over the period of time wherein the cumulative release is between 25% and 75%, preferably, between 25% and 90% by total weight of the drug in the dosage form.

The term "substantially envelops" means that the dosage form, layer, or element in question substantially englobes, coats or covers the material in question. In a preferred embodiment, the dosage form, layer, or element in question envelops more than about 30%, more preferably more than about 50%, more preferably still more than about 75%, and most preferably about 100%, of the material in question, based on the total surface area of the material in question.

### DESCRIPTION OF EMBODIMENTS

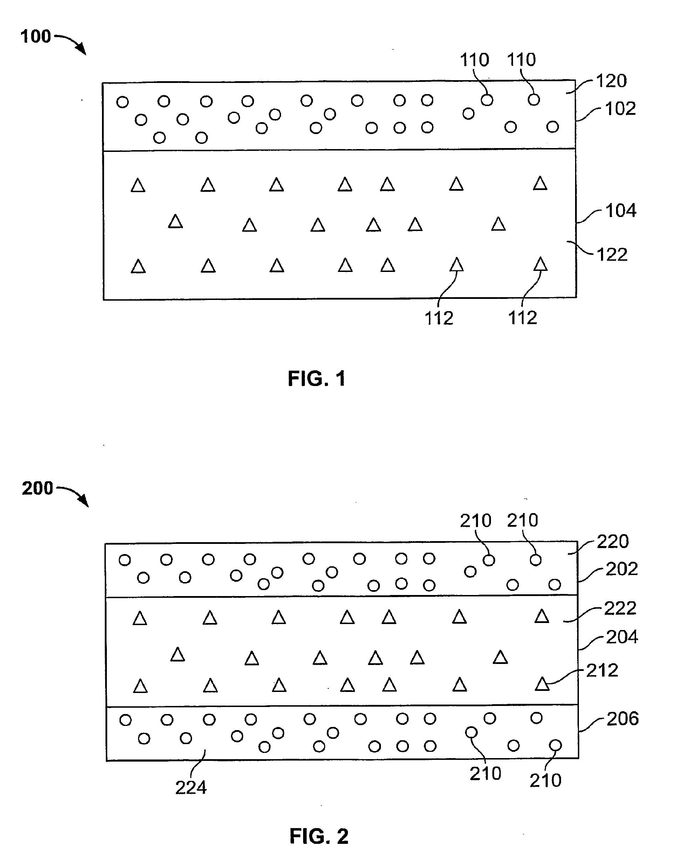

Figure 1 shows sustained release dosage form 100 according to the invention. Included in sustained release dosage form 100 are delay layer 102, second layer 104, microencapsulated drug 110, and non-microencapsulated drug 112. Delay layer 102 is located adjacent to second layer 104. Delay layer 102 comprises polymeric matrix 120 and microencapsulated drug 110. Second layer 104 comprises polymeric matrix 122 and non-microencapsulated 112. In operation, delay layer 102 acts to delay release of drug from sustained release dosage form 100 by delaying release of non-microencapsulated drug 112 from the interface defined by the contacting portion of delay layer 102 and second layer 104. This may occur through a combination of erosion of the delay layers and diffusion of drug through the delay layers. Release of microencapsulated drug 110 is further delayed due to the effect of microencapsulation. In general (meaning that this mechanism may explain operation of multiple embodiments according to the invention), when microencapsulated drug 110 comes in contact with media such as GI fluids (by means of either being in the surface of dosage form 100, erosion of polymeric matrix 120 or diffusion through polymeric matrix 120), the microencapsulating material slowly dissolves in a controlled fashion leaving pores in the coating layer and thereby releasing the drug through these pores. The sum of the effects of the delay layers and the microencapsulation of the drug results in an ascending release rate

Figure 2 shows sustained release dosage form 200 according to the invention. Included in sustained release dosage form 200 are delay layer 202, second layer 204, second delay layer 206, microencapsulated drug 210, non-microencapsulated drug 212, first polymer matrix 220, second polymer matrix 222, and third polymer matrix 224. Delay layer 202 is located adjacent to second layer 204. Delay layer 202 comprises polymeric matrix 220 and microencapsulated drug 210. Second layer 204 comprises polymeric matrix 222 and non-microencapsulated drug 212. Second delay layer 206 is located adjacent to second layer 204 on a portion of dosage form 200 opposite from delay layer 202. Second delay layer 206 comprises polymeric matrix 224 and microencapsulated drug 210.

In operation, when dosage form 200 is dosed to a patient in need thereof, delay layer 202 and second delay layer 206 act to delay release of drug from dosage form 200 by delaying release of non-microencapsulated drug 212 from the interfaces defined by the portion of second layer 204 that contacts delay layer 202 and second delay layer 206. This may occur through a combination of erosion of the delay layers and diffusion of drug through the delay layers. Release of microencapsulated drug 210 is further delayed due to the effect of microencapsulation. Dosage form 200 comprises a polymeric matrix dosage form, which is porous in nature. When the polymeric matrix comes in contact with water or aqueous gastrointestinal fluid, polymer in the polymeric matrix absorbs the water or fluid and undergoes swelling or hydration. This process leads to the relaxation of the polymeric chain and subsequently, the chains disentangle, and as a result of increased distance of separation between the chains, the drug diffuses. As discussed above, a combination of erosion, diffusion, and swelling of the polymeric matrix plus dissolution out of the microcapsules are the various methods through which this system control drug release. By balancing the release impact of the polymeric matrix and the microencapsulation material, an ascending release profile can be obtained.

When the inventive dosage forms, exemplified here as dosage form 200 but potentially applicable to other dosage forms as well, are compared to the classic polymeric matrix (Comparative Example 1, for instance) one can appreciate three very distinctive components to the release profile: (a) there is a reduced initial burst. While not wishing to be bound by a particular mechanism. this effect can possibly be attributed to a combination of the microencapsulated drug present in the outer layers and the composition of these outer layers (large amount of a less hydrophilic polymer that confers to the delay layers a more hydrophobic nature which may prevent an initial sudden swelling and drug release); (b) there is a peak in the profile that characterizes it as an "ascending profile." This effect is probably attained by a combination of the release of the non-microencapsulated drug over time and the micro encapsulated drug through the polymeric matrix by an erosion and diffusion mechanism; and (c) there is a shut down release at the end of the profile. This effect may be due to the release of the microencapsulated drug as well as polymer diffusion and erosion.

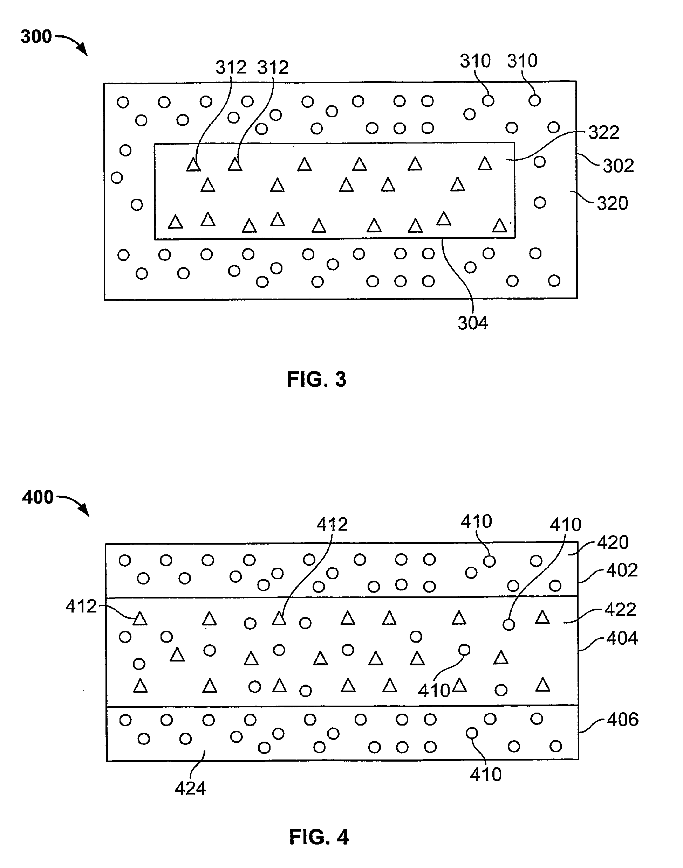

Figure 3 shows sustained release dosage form 300 according to the invention. Included in sustained release dosage form 300 are delay layer 302, second layer 204, microencapsulated drug 310, non-microencapsulated drug 312, first polymer matrix 320, and second polymer matrix 322. Delay layer 302 substantially envelops second layer 304. Delay layer 302 comprises polymeric matrix 320 and microencapsulated drug 310. Second layer 304 comprises polymeric matrix 322 and non-microencapsulated drug 312. In operation, when dosage form 300 is dosed to a patient in need thereof, delay layer 302 acts to delay release of drug from dosage form 300 by delaying release of non-microencapsulated drug 312 from second layer 306. This may occur through a combination of erosion of the delay layers and diffusion of drug through the delay layers. The release mechanisms applicable to dosage form 300 have been discussed above in the discussion of dosage form 200.

Figure 4 shows sustained release dosage form 400 according to the invention. Included in sustained release dosage form 400 are delay layer 402, second layer 404, second delay layer 406, microencapsulated drug 410, non-microencapsulated drug 412, first polymer matrix 420, second polymer matrix 422, and third polymer matrix 424. Delay layer 402 is located adjacent to second layer 404. Delay layer 402 comprises polymeric matrix 420 and microencapsulated drug 410. Second layer 404 comprises polymeric matrix 422, microencapsulated drug 410, and non-microencapsulated drug 412. Second delay layer 406 is located adjacent to second layer 404 on a portion of dosage form 400 opposite from delay layer 402. Second delay layer 406 comprises polymeric matrix 424 and microencapsulated drug 410. In operation, when dosage form 400 is dosed to a patient in need thereof, delay layer 402 and second delay layer 406 act to delay release of drug from dosage form 400 by delaying release of non-microencapsulated drug 412 from the interfaces defined by the portion of second layer 404 that contacts delay layer 402 and second delay layer 406. This may occur through a combination of erosion of the delay layers and diffusion of drug through the delay layers. The release mechanisms applicable to dosage form 400 have been discussed above in the discussion of dosage form 200.

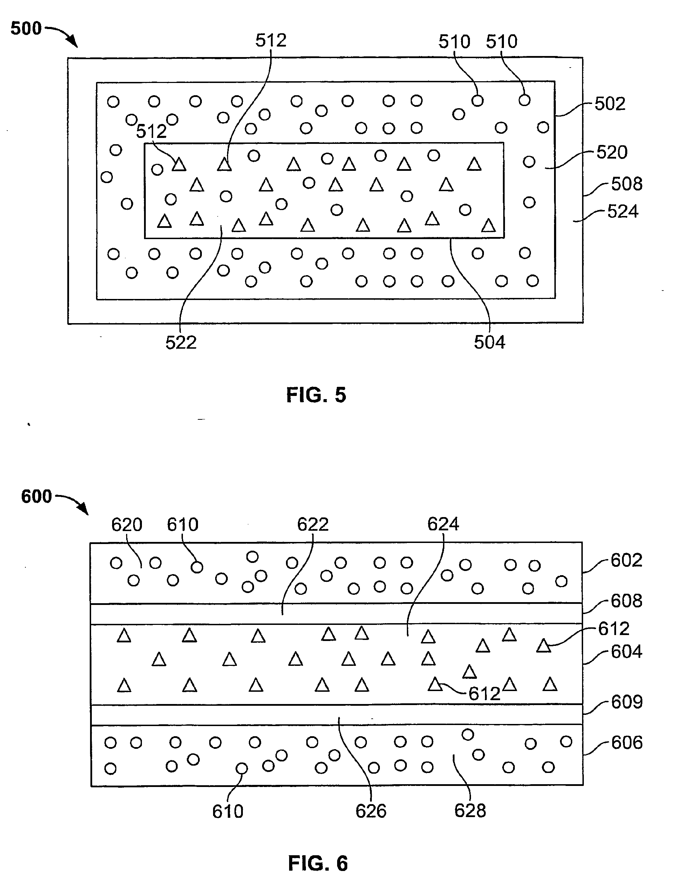

Figure 5 shows sustained release dosage form 500 according to the invention. Included in sustained release dosage form 500 are delay layer 502, second layer 504, outer delay layer 508, microencapsulated drug 510, non-microencapsulated drug 512, first polymer matrix 520, second polymer matrix 522, and third polymer matrix 524. Outer delay layer 508 substantially envelops delay layer 502. Delay layer 502 substantially envelops second layer 504. Outer delay layer 508 comprises polymeric matrix 524. Delay layer 502 comprises first polymeric matrix 520 and microencapsulated drug 510. Second layer 504 comprises polymeric matrix 522 and non-microencapsulated drug 512. In operation, when dosage form 500 is dosed to a patient in need thereof, delay layer 502 acts to delay release of drug from dosage form 500 by delaying release of non-microencapsulated drug 512 from second layer 504. This may occur through a combination of erosion of the delay layers and diffusion of drug through the delay layers. Outer delay layer 508 further delays release, and aids to reduce any initial burst or initial under-controlled release of drug from dosage form 500. The release mechanisms applicable to dosage form 500 have been discussed above in the discussion of dosage form 200.

Figure 6 shows sustained release dosage form 600 according to the invention. Included in sustained release dosage form 600 are delay layer 602, second layer 604, second delay layer 606, third delay layer 608, fourth delay layer 609, microencapsulated drug 610, non-microencapsulated drug 612, first polymer matrix 620, second polymer matrix 622, third polymer matrix 624, fourth polymer matrix 626, and fifth polymer matrix 628. Delay layer 602 is located adjacent to third delay layer 608. Delay layer 602 comprises first polymeric matrix 620 and microencapsulated drug 610. Third delay layer 608 is located adjacent to second layer 604. Third delay layer 608 comprises polymeric matrix 622. Second layer 604 comprises third polymeric matrix 624 and non-microencapsulated drug 612. Fourth delay layer 609 is located adjacent to second layer 604 on a portion of dosage form 600 opposite from delay layer 602. Fourth delay layer 606 comprises fourth polymeric matrix 626. Second delay layer 606 is located adjacent to fourth delay layer 609 on a side of fourth delay layer 609 opposite from second layer 604. Second delay layer 606 comprises fifth polymeric matrix 628 and microencapsulated drug 610. In operation, when dosage form 600 is dosed to a patient in need thereof, delay layer 602 second delay layer 606, third delay layer 608, and fourth delay layer 609 act to delay release of drug from dosage form 600 by delaying release of non-microencapsulated drug 612. This may occur through a combination of erosion of the delay layers and diffusion of drug through the delay layers. In particular, the composition and thickness of third delay layer 608 and fourth delay layer 609 can be chosen to reduce any initial release or burst of non-microencapsulated drug 612 from dosage form 600. The release mechanisms applicable to dosage form 600 have been discussed above in the discussion of dosage form 200.

The inventive dosage forms can be made using conventional techniques, known to one of skill. Such techniques include, but are not limited to, those disclosed in United States Patent 5,980,942 to Katzhendler et al. Additional information relating to manufacturing dosage forms according to the invention may be found, inter alia, in Development of a Controlled Release Matrix Tablet Containing a Water-Soluble Drug Utilizing Hypromellose and Ethylcellulose. Tina Dasbach, Pushpa Inbasekaran, and Karen Balwinski .The Dow Chemical Company, Midland, MI 48674; The Effect of Process Conditions on Various Sustained Release Formulations During Wet Granulation. Pushpa Inbasekaran and Karen Balwinski.The Dow Chemical Company, Midland, MI 48674; Direct Compression of Sustained-Release Hydrophilic Matrix Tablets Containing Hypromellose and MCC: Effects of a Lubricant T. D. Cabelka Technical Service and Development for METHOCEL Cellulose Ethers Larkin Laboratory, The Dow Chemical Company, Midland, MI 48674 USA; Lab-Scale to Full Production Scale Evaluation of a Controlled-Release Formulation Based on Hypromellose and Manufactured Using Roll Compaction Technology Paul Sheskey1, Kerry Pacholke1, Gary Sackett2, and Larry Maher, 1 The Dow Chemical Company Larkin Laboratory Midland, MI 48674. 2 The Vector Corporation Marion, IA 52302; The Utility of Hydroxypropyl Methylcellulose as a Porosity Modifier in an Ethylcellulose Compression Coating Douglas K. Pollock and Karen M. Balwinski Presented at the 27th International Symposium on Controlled Release of Bioactive Materials Paris, France, July 7-13, 2000 The Dow Chemical Company Larkin Laboratory, Midland, MI 48674 USA.

The amount of drug incorporated in the dosage forms of the present invention varies depending on the particular drug, the therapeutic indication and the desired administration period, e.g., every 12 hours, every 24 hours, etc. Depending on the dose of drug desired to be administered, one or more of the dosage forms may be administered.

There are numerous clinical situations and drug therapies that could be improved with the use of dosage forms that provide a sustained and ascending release rate over an extended time period. Exemplary dosage forms, as disclosed herein, comprise CNS-acting drugs and cardiovascular-acting drugs. It will be appreciated by persons of skill in the art that the invention is applicable to many other types of drugs and drug therapies. Examples of suitable types of drugs include, but are not limited to, antiinfectives, analgesics, anesthetics, antiarthritics, antiasthmatics, anticonvulsants, antidepressants, antidiabetics, antidiarrheals, antihistamines, antiinflammatories, antimigraines, antineoplastics, antiparkinsonisms, antipruritics, antipsychotics, antipyretics, antispasmodics, anticholinergics, sympathomimetics, calcium channel blockers, beta blockers, antiarrythmics, antihypertensives, ACE inhibitors, diuretics, vasodilators, decongestants, hormones, hypnotics, immunosuppresives, parasympathomimetics, prostaglandins, proteins, peptides, sedatives and tranquilizers.

Dosage forms according to the invention can be administered for treatment of a number of indications, including but not limited to CNS and cardiovascular indications.

The following examples are illustrative of the present invention, and the examples should not be considered as limiting the scope of the invention in any way, as these examples, and other equivalents thereof, will become apparent to those versed in the art in the light of the present disclosure and the accompanying claims.

### EXAMPLES

### EXAMPLE 1

Approximately five percent of label claim was present in the dosage form as microencapsulated drug in the delay layers of the dosage form and approximately 95% of label claim was present as free drug in the second layer. This configuration was targeted to provide an ascending release rate

Microencapsulation of topiramate: Fifty grams of topiramate were weighed and transferred to a beaker. Six grams of Kollicoat 30DEMM were added dropwise into the topiramate and mixed with a spatula for 5 minutes. The wet mass was passed through an 8 mesh screen and allowed to air dry for 72 hrs. The dried mass was passed through a 12 mesh screen. The granulation was placed on a tray and 6g of Kollidon 30DEMM were sprayed onto the granules and placed in an oven at 28C, ambient humidity. The coating process was repeated twice again, each time adding approx 6g of Kollidon 30DEMM. After the third coating, the granules were left in the oven overnight and the dry granules were passed through a 8 mesh screen

Preparation of delay layers: 230.8 mg of microencapsulated topiramate, as described above, were mixed with 1.3 g of HPMC K100 M Prem CR and 1.3 g of ethyl cellulose. The materials were mixed in a roller mixer (U.S. Stoneware Jar Mill (Model 764 AVM)) for 30 min.

Preparation of second layer: 2.8 g of topiramate was mixed with 1.9 g of HPMC K15M Prem CR and 1.7 g of Ethyl cellulose and mixed in a roller mixer (U.S. Stoneware Jar Mill (Model 764 AVM)) for 30 min. 3.4 g of PEG 3350 and 30 mg of black ferric oxide were added and mixed for 20 min in the roller mill. 30 mg of Magnesium stearate was added and mixed for 30 seconds

Compression of tablets: 32.5 mg of the delay layer composition was weighed and added to the die cavity of a 3/8" round concave punch set. The delay layer was lightly tamped. 335 mg of second layer composition was added to the tamped delay layer and lightly tamped. 32.5 mg of delay layer composition was added and the tablet was compressed using a compression force of 2 tons in a Carver press.

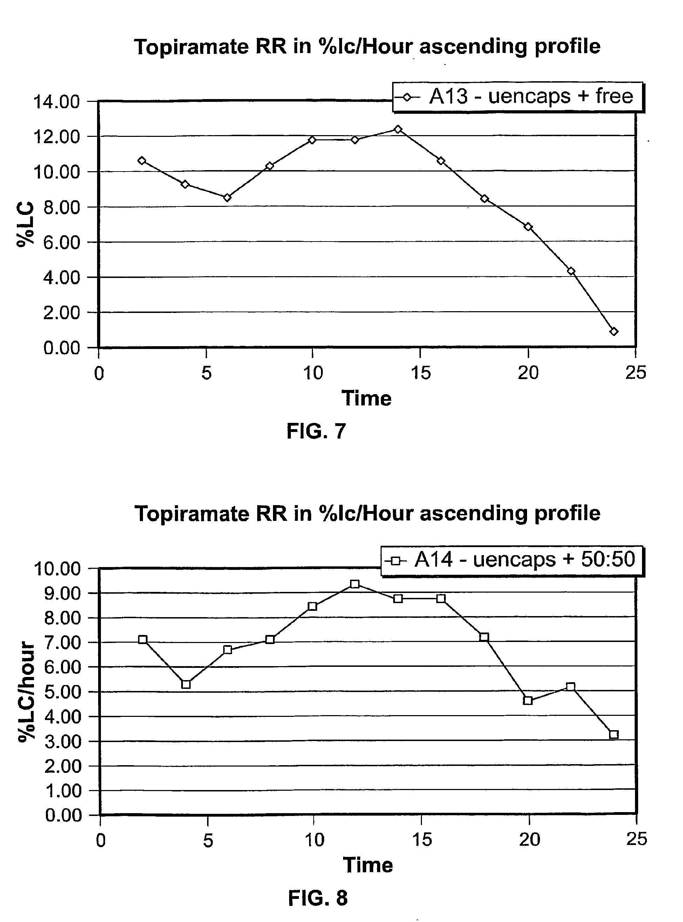

Determination of drug release: The compressed tablets were analyzed for drug release in water using a USP type 2 apparatus. The results are presented in Figure 7.

### EXAMPLE 2:

Approximately five percent of label claim was present as microencapsulated drug in the delay layers of the dosage form and approximately 95% of label claim was present in the second layer 2 as 42.5% non-microencapsulated drug and 42.5% microencapsulated drug. This configuration was targeted to provide an ascending release rate

The microencapsulated topiramate was prepared as described in Example 1. The delay layer composition was prepared as described in Example 1.

Preparation of second layer: 1.4 g of topiramate was mixed with 1.4 g of microencapsulated topiramate (prepared as described in Example 1), 1.9 g of HPMC K15M Prem CR and 1.7 g of Ethyl cellulose and were mixed in a roller mixer (U.S. Stoneware Jar Mill (Model 764 AVM)) for 30 min. 3.4 g of PEG 3350 and 30 mg of black ferric oxide were added and mixed for 20 min in a roller mill. 30 mg of Magnesium stearate was added and mixed for 30 seconds

The compressed tablet was prepared according to the procedure in Example 1, and using the delay composition from Example 1 and the second layer composition prepared above.

The results are presented in Figure 8.

### EXAMPLE 3:

67% of label claim is present as microencapsulated drug in the inner core and 33% of label claim is present as microencapsulated drug in the inner layer. The outer end caps are drug-free. This configuration provides an ascending release profile.

### STEP 1: MICROENCAPSULATION OF PALIPERIDONE

Batch #1

1 gm of paliperidone was weighed into a beaker along with 2 gm of HPMC K100 Prem. Approximately 1.4 gm of an Ethanol/Kollicoat EMM 30D (83/17 wt./wt.) mixture was added to granulate the mixture.

Batch #2

6 gm of paliperidone was weighed into a beaker along with 2 gm of HPMC K100 Prem. Approximately 1.8 gm of an Ethanol/Kollicoat EMM 30D (75/25 wt./wt.) mixture was added to granulate the mixture.

Batch #3

Approximately 4.8 gm of a Kollicoat EMM 30D was added to 1.35 gm of Batch #2 to granulate the mixture.

**STEP 2: PREPARATION OF OUTER LAYER, INNER DRUG LAYER AND INNER CORE**

| **Material ID** | **Outer Layer** | **Inner Layer** | **Inner Core** |
|---|---|---|---|
| Batch#1 | - | - | - 20.47% |
| Batch#2 | - | - | - |
| Batch#3 | - | 0.84% | - |
| HPMC K15M Prem | - | 36.50% | - |
| HPMC K100M Prem | 50% | - | 33.33% |
| Ethyl Cellulose (w/ St. Acid) | 50% | 27.36% | - |
| PEG 8000K | - | 35.00% | - |
| Ferric oxide | - | 0.30% | - |
| Crospovidone | - | - | 46.20% |

### STEP 3: CORE COMPRESSION

30 mg of inner core layer is weighed and added to the die cavity of a 5/32" round concave punch set. A compression force of 1 ton is applied on a Carver press to yield a single layer inner core.

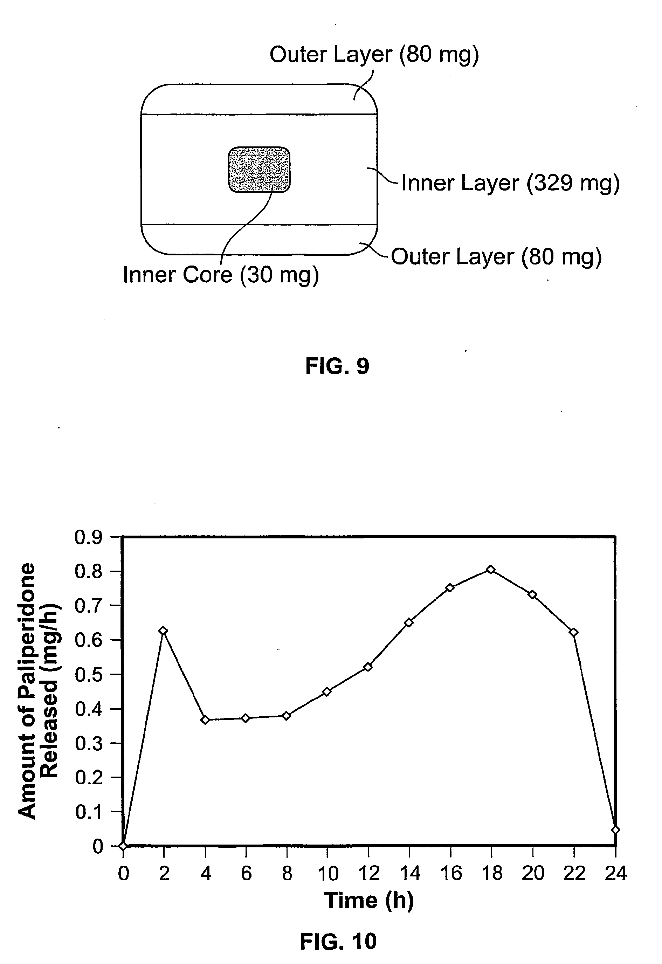

80 mg of outer layer is weighed and added to the die cavity of a 3/8" round concave punch set. The layer is lightly tamped. 329 mg of the inner layer is weighed. Part of the inner drug layer is added to the tamped outer layer and the inner compressed core is placed in the center of the die cavity. The remaining inner drug layer granulation is added to the die cavity and is lightly tamped. 80 mg of outer layer is weighed and added to the die cavity and the trilayer core with an encapsulated inner core is compressed using a compression force of 2 tons on a Carver press. The core layout is illustrated in Figure 9.

### STEP 4: DETERMINATION OF DRUG RELEASE

The compressed cores from Step 3 are analyzed for drug release in AGF using USP Type II apparatus (UV analysis). The results are shown in Figure 10.

### EXAMPLE 4

67% of label claim is present as microencapsulated drug in the inner core and 33% of label claim is present as microencapsulated drug in the inner layer. The outer end caps are drug-free. This configuration provides an ascending release profile.

### STEP 1: MICROENCAPSULATION OF RISPERIDONE

Batch #1

1 gm of risperidone was weighed into a beaker along with 2 gm of HPMC K100 Prem. Approximately 1.4 gm of an Ethanol/Kollicoat EMM 30D (83/17 wt./wt.) mixture was added to granulate the mixture.

Batch #2

6 gm of risperidone was weighed into a beaker along with 2 gm of HPMC K100 Prem. Approximately 1.8 gm of an Ethanol/Kollicoat EMM 30D (75/25 wt./wt.) mixture was added to granulate the mixture.

Batch #3

Approximately 4.8 gm of a Kollicoat EMM 30D was added to 1.35 gm of Batch #2 to granulate the mixture.

### STEP 2: PREPARATION OF OUTER LAYER, INNER DRUG LAYER AND INNER CORE

| **Material ID** | **Outer Layer** | **Inner Layer** | **Inner Core** |
|---|---|---|---|
| | | | |
| Batch#1 | - | - | 20.47% |
| Batch#2 | - | - | - |
| Batch#3 | - | 0.84% | - |
| HPMC K15M Prem | - | 36.50% | - |
| HPMC K100M Prem | 50% | - | 33.33% |
| Ethyl Cellulose (w/ St. Acid) | 50% | 27.36% | - |
| PEG 8000K | - | 35.00% | - |
| Ferric oxide | - | 0.30% | - |
| Crospovidone | - | - | 46.20% |

### STEP 3: CORE COMPRESSION

The compressed tablet was prepared according to the procedure in Example 3, and using the core layer composition from Example 3

### COMPARATIVE EXAMPLE 1:

One hundred percent of label claim of the drug was in a matrix core. This configuration was found to provide an approximately zero order release rate, as described below.

28.6 g of topiramate was mixed with 21.5 g of HPMC K100M Prem and 14.3 g of ethyl cellulose and 35.14 g of Polyetheylene glycol 3350. The components were mixed in a roller mixer (U.S. Stoneware Jar Mill (Model 764 AVM)) for 30 min. 0.5 g of Mg stearate is added and mixed for 30 sec.

350 mg of this blend was compressed at 2 tons using a 3/8" tooling set on a Carver press.

The compressed tablets were analyzed for drug release in water using a USP type 2 apparatus.

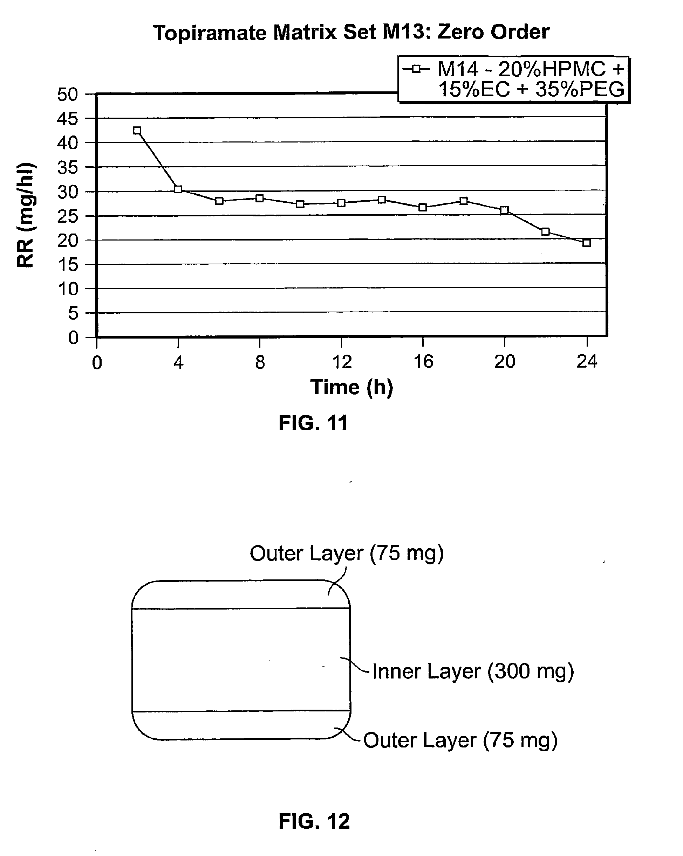

The results are presented in Figure 11.

### COMPARATIVE EXAMPLE 2:

100 % of label claim is present as microencapsulated drug in the inner layer. The outer end caps are drug-free. This configuration provides a zero-order release profile.

### STEP 1: PREPARATION OF OUTER LAYER AND INNER DRUG LAYER

| **Material ID** | **Outer Layer** | **Inner Layer** |
|---|---|---|
| Batch#1 | - | 0.52% |
| Batch#2 | - | 0.69% |
| Batch#3 | - | - |
| HPMC K15M Prem | - | 36.25% |
| HPMC K100M Prem | 50% | - |
| Ethyl Cellulose | 50% | 27.20% |
| PEG 8000K - | | 34.74% |
| Ferric oxide | - | 0.30% |
| Crospovidone - | | - |
| Magnesium Stearate | | 0.30% |

### STEP 2: CORE COMPRESSION

75 mg of outer layer is weighed and added to the die cavity of a 3/8" round concave punch set. The layer is lightly tamped. 300 mg of the inner layer is weighed and added to the tamped outer layer. The granulation is lightly tamped. 75 mg of outer layer is weighed and added to the die cavity and the trilayer core is compressed using a compression force of 3 tons on a Carver press. The core layout is shown in Figure 12.

### STEP 3: DETERMINATION OF DRUG RELEASE

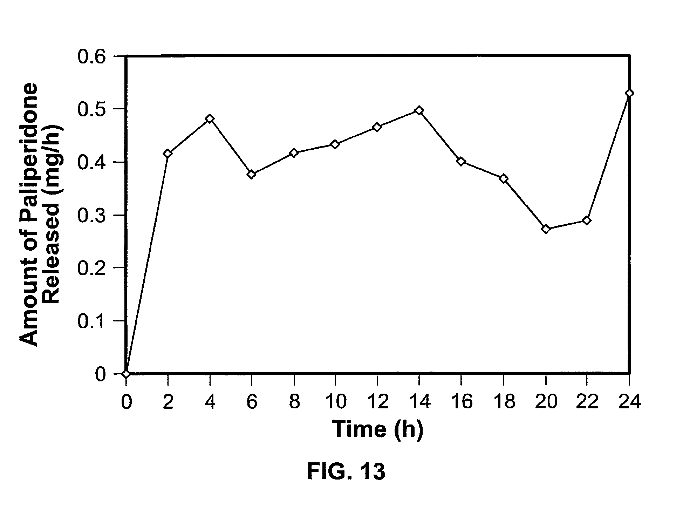

The compressed cores from Step 2 are analyzed for drug release in AGF using USP Type II apparatus (UV analysis). The results are shown in Figure 13.

## Claims

1. A dosage form for sustained release of a drug comprising:
a delay layer comprising
(i) a polymeric matrix, and
(ii) microencapsulated drug, wherein the delay layer is substantially free of non-microencapsulated drug; and
a second layer comprising
(iii) a polymeric matrix, and
(iv) non-microencapsulated drug matrix;
wherein the second layer is located adjacent to the delay layer.

2. The dosage form of claim 1, wherein the ratio of the weight of microencapsulated drug present in the delay layer to the weight of non-microencapsulated drug present in the second layer ranges from 0.01:1 to 10:1

3. The dosage form of claim 1, wherein the second layer further comprises microencapsulated drug.

4. The dosage form of claim 1, further comprising a sub-layer disposed between the delay and second layer.

5. The dosage form of claim 1, further comprising a second delay layer comprising a polymeric matrix material and microencapsulated drug, wherein the second delay layer is substantially free of non-microencapsulated drug; and
wherein the second delay layer is located adjacent to the second layer on a portion of the dosage form opposite from the delay layer.

6. The dosage form of claim 1, wherein the delay layer substantially envelops the second layer.

7. The dosage form of claim 1, wherein the drug comprises topiramate, risperidone, or paliperidone.

8. The dosage form of claim 7, wherein the topiramate is present in an amount ranging from 10 to 1000 mg.

9. The dosage form of claim 7, wherein the risperidone is present in an amount ranging from 1 to 15 mg.

10. The dosage form of claim 7, wherein the paliperidone is present in an amount ranging from 1 to 15 mg.

11. The dosage form of claim 1, wherein the microencapsulated drug is encapsulated by microencapsulation materials that comprise proteins, polysaccharides, starches, waxes, fats, natural and synthetic polymers, resins, or combinations thereof.

12. A method of making a dosage form for sustained release of a drug comprising:
providing a delay layer comprising
(v) a polymeric matrix, and
(vi) microencapsulated drug, wherein the delay layer is substantially free of non-microencapsulated drug; and
providing a second layer comprising
(vii) a polymeric matrix, and
(viii) non-microencapsulated drug matrix; and
locating the second layer adjacent to the delay layer.

13. The method of claim 12, wherein the ratio of the weight of microencapsulated drug present in the delay layer to the weight of non-microencapsulated drug present in the second layer ranges from 0.01:1 to 10:1

14. The method of claim 12, wherein the second layer further comprises microencapsulated drug.

15. The method of claim 12, further comprising providing a sub-layer disposed between the delay and second layer.

16. The method of claim 12, further comprising
providing a second delay layer comprising a polymeric matrix material and microencapsulated drug, wherein the second delay layer is substantially free of non-microencapsulated drug; and wherein
locating the second delay layer adjacent to the second layer on a portion of the dosage form opposite from the delay layer.

17. The method of claim 12, wherein the delay layer substantially envelops the second layer.

18. The method of claim 12, wherein the drug comprises topiramate, risperidone, or paliperidone.

19. The method of claim 18, wherein the topiramate is present in an amount ranging from 10 to 1000 mg.

20. The method of claim 18, wherein the risperidone is present in an amount ranging from 1 to 15 mg.

21. The method of claim 18, wherein the paliperidone is present in an amount ranging from 1 to 15 mg.

22. The method of claim 12, wherein the microencapsulated drug is encapsulated by microencapsulation materials that comprise proteins, polysaccharides, starches, waxes, fats, natural and synthetic polymers, resins, or combinations thereof.

## Patentansprüche

1. Dosierungsform mit verzögerter Freisetzung eines Arzneimittels umfassend
eine Verzögerungsschicht umfassend
(i) eine polymere Matrix, und
(ii) ein mikroverkapseltes Arzneimittel, wobei die Verzögerungsschicht im wesentlichen frei von nicht-mikroverkapseltem Arzneimittel ist und
eine zweite Schicht umfassend
(iii) eine polymere Matrix und
(iv) eine nicht-mikroverkapselte Arzneimittelmatrix,
wobei die zweite Schicht benachbart der Verzögerungsschicht lokalisiert ist.

2. Dosierungsform nach Anspruch 1, bei der das Verhältnis des Gewichts des mikroverkapselten Arzneimittels, das in der Verzögerungsschicht vorhanden ist, zu dem Gewicht des nicht-mikroverkapselten Arzneimittels, das in der zweiten Schicht vorhanden ist, von 0,01 : 1 bis 10:1 reicht.

3. Dosierungsform nach Anspruch 1, bei der die zweite Schicht des weiteren mikroverkapseltes Arzneimittel umfaßt.

4. Dosierungsform nach Anspruch 1, des weiteren umfassend eine Teilschicht, die zwischen der Verzögerungsschicht und der zweiten Schicht angeordnet ist.

5. Dosierungsform nach Anspruch 1, des weiteren umfassend eine zweite Verzögerungsschicht, die ein polymeres Matrixmaterial und mikroverkapseltes Arzneimittel aufweist, wobei die zweite Verzögerungsschicht im wesentlichen frei von nicht-mikroverkapseltem Arzneimittel ist, und wobei die zweite Verzögerungsschicht benachbart der zweiten Schicht auf einem Teil der Dosierungsform gegenüber der Verzögerungsschicht lokalisiert ist.

6. Dosierungsform nach Anspruch 1, bei der die Verzögerungsschicht im wesentlichen die zweite Schicht umhüllt.

7. Dosierungsform nach Anspruch 1, bei der das Arzneimittel Topiramat, Risperidon:oder Paliperidon:umfaßt.

8. Dosierungsform nach Anspruch 7, bei der das Topiramat in einer Menge im Bereich von 10 bis 1000 mg vorhanden ist.

9. Dosierungsform nach Anspruch 7, bei der das Risperidon in einer Menge im Bereich von 1 bis 15 mg vorhanden ist.

10. Dosierungsform nach Anspruch 7, bei der das Paliperidon in einer Menge im Bereich von 1 bis 15 mg vorhanden ist.

11. Dosierungsform nach Anspruch 1, bei der das mikroverkapselte Arzneimittel mit Mikroverkapselungsmaterialien eingekapselt ist, die Proteine, Polysaccharide, Stärken, Wachse, Fette, natürliche und **synthetische Polymere, Harze oder deren Kombinationen umfassen.**

12. Verfahren zur Herstellung einer Dosierungsform mit verzögerter Freisetzung eines Arzneimittels umfassend
Bereitstellung einer Verzögerungsschicht umfassend
(i) eine polymere Matrix, und
(ii) ein mikroverkapseltes Arzneimittel, wobei die Verzögerungsschicht im wesentlichen frei von nicht-mikroverkapseltem Arzneimittel ist und
Bereitstellung einer zweiten Schicht umfassend
(iii) eine polymere Matrix und
(iv) eine nicht-mikroverkapselte Arzneimittelmatrix, und
Lokalisierung der zweite Schicht benachbart der Verzögerungsschicht.

13. Verfahren nach Anspruch 12, bei dem das Verhältnis des Gewichts des mikroverkapselten Arzneimittels, das in der Verzögerungsschicht vorhanden ist, zu dem Gewicht des nicht-mikroverkapselten Arzneimittels, das in der zweiten Schicht vorhanden ist, von 0,01 : 1 bis 10 : 1 reicht.

14. Verfahren nach Anspruch 12, bei der die zweite Schicht des weiteren mikroverkapseltes Arzneimittel umfaßt.

15. Verfahren nach Anspruch 12, des weiteren umfassend eine Teilschicht, die zwischen der Verzögerungsschicht und der zweiten Schicht angeordnet ist.

16. Verfahren nach Anspruch 12, des weiteren umfassend
Bereitstellung einer zweiten Verzögerungsschicht, die ein polymeres Matrixmaterial und mikroverkapseltes Arzneimittel aufweist, wobei die zweite Verzögerungsschicht im wesentlichen frei von nicht-mikroverkapseltem Arzneimittel ist, und
Lokalisierung der zweiten Verzögerungsschicht benachbart der zweiten Schicht auf einem Teil der Dosierungsform gegenüber der Verzögerungsschicht.

17. Verfahren nach Anspruch 12, bei der die Verzögerungsschicht im wesentlichen die zweite Schicht umhüllt.

18. Verfahren nach Anspruch 12, bei der das Arzneimittel Topiramat, Risperidon: oder Paliperidon umfaßt.

19. Verfahren nach Anspruch 18, bei der das Topiramat in einer Menge im Bereich von 10 bis 1000 mg vorhanden ist.

20. Verfahren nach Anspruch 18, bei der das Risperidon in einer Menge im Bereich von 1 bis 15 mg vorhanden ist.

21. Verfahren nach Anspruch 18, bei der das Paliperidon in einer Menge im Bereich von 1 bis 15 mg vorhanden ist.

22. Verfahren nach Anspruch 12, bei der das mikroverkapselte Arzneimittel mit Mikroverkapselungsmaterialien eingekapselt ist, die Proteine, Polysaccharide, Stärken, Wachse, Fette, natürliche und synthetische Polymere, Harze oder deren Kombinationen umfassen.

## Revendications

1. - Forme posologique pour la libération entretenue d'un médicament comprenant :
- une couche retard comprenant
(i) une matrice polymère ; et
(ii) un médicament microencapsulé, la couche retard étant sensiblement exempte de médicament non-microencapsulé ; et
- une seconde couche comprenant
(iii) une matrice polymère ; et
(iv) une matrice de médicament non microencapsulé ; la seconde couche étant située adjacente à la couche de retard.

2. - Forme posologique selon la revendication 1, dans laquelle le rapport du poids de médicament microencapsulé présent dans la couche retard au poids de médicament non microencapsulé présent dans la seconde couche se situe dans la plage de 0,01:1 à 10:1.

3. - Forme posologique selon la revendication 1, dans laquelle la seconde couche comprend en outre un médicament microencapsulé.

4. - Forme posologique selon la revendication 1, comprenant en outre une sous-couche disposée entre la couche retard et la seconde couche.

5. - Forme posologique selon la revendication 1, comprenant en outre une seconde couche retard comprenant une matière de matrice polymère et un médicament microencapsulé, dans laquelle la seconde couche retard est sensiblement exempte de médicament non-microencapsulé ; et dans laquelle la seconde couche retard est située adjacente à la seconde couche sur une partie de la forme posologique opposée à la couche retard.

6. - Forme posologique selon la revendication 1, dans laquelle la couche retard enveloppe de façon substantielle la seconde couche.

7. - Forme posologique selon la revendication 1, dans laquelle le médicament comprend le topiramate, la rispéridone ou la palipéridone.

8. - Forme posologique selon la revendication 7, dans laquelle le topiramate est présent dans une quantité se situant dans la plage de 10 à 1000 mg.

9. - Forme posologique selon la revendication 7, dans laquelle la rispéridone est présente dans une quantité se situant dans la plage de 1 à 15 mg.

10. - Forme posologique selon la revendication 7, dans laquelle la palipéridone est présente dans une quantité se situant dans la plage de 1 à 15 mg.

11. - Forme posologique selon la revendication 1, dans laquelle le médicament microencapsulé est encapsulé par des matières de microencapsulation qui comprennent les protéines, les polysaccharides, les amidons, les cires, les graisses, les polymères naturels et synthétiques, les résines ou leurs combinaisons.

12. - Procédé de fabrication d'une forme posologique pour libération entretenue d'un médicament comprenant les opérations consistant à :
- prendre une couche retard comprenant
(v) une matrice polymère ; et
(vi) un médicament microencapsulé, la couche retard étant sensiblement exempte de médicament non microencapsulé ; et
- prendre une seconde couche comprenant
(vii) une matrice polymère ; et
(viii) une matrice de médicament non-microencapsulé ; et
- positionner la seconde couche adjacente à la couche retard.

13. - Procédé selon la revendication 12, dans lequel le rapport du poids de médicament microencapsulé présent dans la couche retard au poids du médicament non microencapsulé présent dans la seconde couche se situe dans la plage de 0,01:1 à 10:1.

14. - Procédé selon la revendication 12, dans lequel la seconde couche comprend en outre un médicament microencapsulé.

15. - Procédé selon la revendication 12, comprenant en outre la disposition d'une sous-couche disposée entre la couche retard et la seconde couche.

16. - Procédé selon la revendication 12, comprenant en outre les opérations consistant à :
- prendre une seconde couche retard comprenant une matière de matrice polymère et un médicament microencapsulé, la seconde couche retard étant sensiblement exempte de médicament non microencapsulé ; et
- positionner la seconde couche retard adjacente à la seconde couche sur une partie de la forme posologique opposée à la couche retard.

17. - Procédé selon la revendication 12, dans lequel la couche retard enveloppe de façon substantielle la seconde couche.

18. - Procédé selon la revendication 12, dans lequel le médicament comprend le topiramate, la rispéridone ou la palipéridone.

19. - Procédé selon la revendication 18, dans lequel le topiramate est présent dans une quantité se situant dans la plage de 10 à 1000 mg.

20. - Procédé selon la revendication 18, dans lequel la rispéridone est présente dans une quantité se situant dans la plage de 1 à 15 mg.

21. - Procédé selon la revendication 18, dans lequel la palipéridone est présente dans une quantité se situant dans la plage de 1 à 15 mg.

22. - Procédé selon la revendication 12, dans lequel le médicament microencapsulé est encapsulé par des matières de microencapsulation qui comprennent les protéines, les polysaccharides, les amidons, les cires, les graisses, les polymères naturels et synthétiques, les résines ou leurs combinaisons.
